Europäisches Patentamt

European Patent Office

Office européen des brevets

(11) Veröffentlichungsnummer: **0 670 338 A2**

(12) **EUROPÄISCHE PATENTANMELDUNG**

(21) Anmeldenummer: **95102629.3**

(22) Anmeldetag: **24.02.95**

(51) Int. Cl.[6]: **C08F 220/04**, C08F 220/12, C08F 2/46, C08F 283/06, C09J 133/00, A61L 25/00

(30) Priorität: **03.03.94 DE 4406978**

(43) Veröffentlichungstag der Anmeldung:
**06.09.95 Patentblatt 95/36**

(84) Benannte Vertragsstaaten:
**AT BE CH DE DK ES FR GB GR IE IT LI LU NL PT SE**

(71) Anmelder: **Lohmann GmbH & Co. KG**
**Irlicher Strasse 55**
**D-56567 Neuwied (DE)**

(72) Erfinder: **Czech, Zbigniew, Dr.**
**Rostocker Strasse 10**
**D-56075 Koblenz (DE)**

(74) Vertreter: **Flaccus, Rolf-Dieter, Dr.**
**Patentanwalt**
**Sperlingsweg 32**
**D-50389 Wesseling (DE)**

(54) **Verfahren zur Herstellung von selbstklebenden Hydrogelen.**

(57) Bei einem zur Herstellung von selbstklebenden Hydrogelen durch radikalische Polymerisation eines photopolymerisationsfähigen, lösemittelfreien Ausgangsgemisches mit Hilfe von Ultraviolettstrahlen im Bereich von 180-400 nm, wobei die zur Polymerisationsauslösung erforderliche Energie durch monochromatische Strahlung eines Lasers zugeführt wird, enthält das polymerisationsfähige, lösemittelfreie Ausgangsgemisch folgende Bestandteile:

A - eine Vinylcarbonsäure
B - ein (Meth)acrylsäurealkylester mit 1 bis 12 Kohlenstoffatomen im Alkylrest
C - ein Polyoxyalkylen mit einer Molekularmasse bis 2000 Dalton, Glycerin oder deren Ester
D - ein hydroxylgruppenhaltiges Amin
E - einen Fotoinitiator.

EP 0 670 338 A2

Die Erfindung betrifft ein Verfahren zur Herstellung von selbstklebenden Hydrogelen durch Polymerisation eines lösemittelfreien Monomerengemisches sowie ihre Verwendung als medizinische Wundauflagen.

Es sind eine Reihe von Hydrogel-Wundauflagen auf dem Markt, die ein hohes Absorptionsvermögen für Wundsekrete aufweisen, so daß ein mehrtägiger Heilungsprozeß der Wunde unter Ausschluß von Bakterien gewährleistet ist.

Derartige Hydrogele weisen jedoch verschiedene Nachteile auf, die ihre praktische Anwendung stark einschränken. Beispielsweise haben solche Hydrogelmaterialien nach der Wundsekretabsorption keine ausreichende mechanische Festigkeit. Sie lösen sich oft bei Körpertemperatur auf und lassen sich dann nicht ruckstandsfrei von der Wunde entfernen. Außerdem haben sie eine ungenügende Transparenz, so daß ein kontinuierliche Beobachtung des Heilungsprozesses ohne Wundauflagenwechsel erschwert ist.

Um diese Nachteile zu beseitigen, wurde bereits versucht, Hydrogele durch eine Polymerisations-Reaktion herzustellen. Die hierfür benötigte Energie wurde dem Ausgangsmonomerengemisch in Form spektraler UV-Strahlen (UV-Lampe) zugeführt.

Beispielsweise beschreibt die DE-PS 35 06 534 eine UV-initiierte Polymerisation von (Meth)acrylamid und Vinylsulfonderivaten, die aber nicht zu flexiblen, für Hydrogele geeigneten Materialien führte.

Weiterhin sind aus der US-A 4 620 954 durch UV-initiierte Polymerisation hergestellt Polymerisate aus N-Vinylpyrrolidon und Phenylethylmethacrylat bekannt. Sie sind zwar transparent, weisen aber keine Hafteigenschaften auf.

In der DE-A 38 25 366 ist ein kontinuierlicher Herstellprozeß eines Acrylatpolymer-Gels beschrieben, bei dem das Bestrahlen der Schicht aus der Monomerlösung mit Lichtenergie erfolgt. Dieser Photopolymerisationsprozeß enthält eine ganze Reihe von Nachteilen wie beispielsweise ein notwendiges Herabsetzen des Gehaltes an gelöstem Sauerstoff in der Monomerlösung unter 1 mg/l oder Aufrechterhalten der Sauerstoffkonzentration in der Gasphase im Inneren einer oberhalb eines bewegten Trägers angeordneten, gasdichten Kammer auf einen Wert von nicht mehr als 1 Vol.-%.

Die US-A-4 189 370 beschreibt ein Polymer-Gel, das durch radikalische Polymerisation von N-Methylolacrylamid mit bifunktionellen Allyl- oder Acrylatderivaten hergestellt wird. Die Polymerisation wird mit Peroxiden oder mit UV-Strahlen gestartet.

In der EP-A 072 213 ist ein wasserhaltiges Copolymer-Gel auf Basis von Acrylamid/(Meth)-acrylsäuresalz mit Kaolin, Talk etc. als flow agent beschrieben.

Die JP 60 149 416 und JP 60 149 411 beschreiben ein Gel bestehend aus polyfunktionellen UV-härtbaren Polyacrylaten und Polyestern.

US-A 4 790 919 umfaßt photopolymerisierte Gele auf Acrylatbasis.

JP 63 081 112 beschreibt ein UV-härtbares Gel, zusammengesetzt aus Urethanacrylat und N-Vinylpyrrolidon.

US-A-4 914 173 beschreibt UV-vernetzbare acrylierte Polyurethan-Gele.

EP-A-327 304 umfaßt elektrisch leitfähige Gele auf Basis von UV-härtbaren Urethanacrylatoligomeren. Zwecks Erreichen der elektrischen Leitfähigkeit wurde als Elektrolyt $MgBr_2$ eingesetzt.

US-A-5 141 973 beschreibt die Herstellungsmethode eines Polyvinylalkohol-Gels. Um ein Gel zu bilden, müssen die PVA-Moleküle physikalisch angeregt werden. Als Anregungsmöglichkeit bietet sich die Laserbestrahlung an.

JP 2 173 102 erwähnt ein Gel, zusammengesetzt aus partiell hydrolysiertem Acrylamid-Polymer. Die Vernetzung des Gels erfolgt mittels UV-Strahlung.

EP-A-396 246 beschreibt ein UV-härtbares System, in dem acryloyloxyfunktionelle, monoacryloyloxyfunktionelle und nicht funktionelle Polydiorganosiloxane eingesetzt werden.

JP 3 192 106 beschreibt UV-härtbare Gele, zusammengesetzt aus hydroxylgruppenhaltigen (Meth)-acrylaten (Urethan(meth)acrylate), ungesättigten Derivaten mit Ethylenstruktur, Harzen und Fotoinitiatoren.

JP 3 192 107 beschreibt UV-härtbare Gele auf Basis von N-substituierten Acrylamiden.

CA-A 2 037 703 beschreibt einen Herstellungsprozeß von Kontaktlinsen, wobei ein Laser zur Vernetzung oder zur Polymerisation verwendet wurde. Die Kontaktlinsen sind auf Basis von Hydroxyethylmethacrylat aufgebaut. Die Polymerisation erfolgt in einer sauerstofffreien oder inerten Atmosphäre.

Der vorliegenden Erfindung liegt die Aufgabe zugrunde, selbstklebende wundsekretabsorbierende Hydrogele anzugeben, welche die vorgenannten Nachteile nicht aufweisen, sowie ein Verfahren zu Ihrer Herstellung aufzuzeigen, wobei die Hydrogele ein hohes Absorptionsvermögen für Wundsekrete, dabei eine ausreichende mechanische Festigkeit nach der Wundsekretabsorption und eine ausreichende Transparenz für eine einwandfreie Wundbeobachtung aufweisen sollen.

Diese Aufgabe wird erfindungsgemäß bei einem Verfahren zur Herstellung von selbstklebenden Hydrogelen durch radikalische Polymerisation eines photopolymerisationsfähigen, lösemittelfreien Ausgangsgemisches mit Hilfe von Ultraviolettstrahlen im Bereich von 180 bis 400 nm, wobei die zur Polymerisationsauslösung erforderliche Energie durch monochromatische Strahlung eines Lasers zugeführt wird, dadurch gelöst, daß das polymerisationsfähige, lösemittelfreie Ausgangsgemisch folgende Bestandteile enthält:

A   - eine Vinylcarbonsäure in 30 bis 60 Gew.-%

B   - ein (Meth)acrylsäurealkylester mit 1 bis 12 Kohlenstoffatomen im Alkylrest in 10 bis 30 Gew.-%

C   - ein Polyoxyalkylen mit einer Molekularmasse bis 2000 Dalton, Glycerin oder deren Ester in 10 bis 40 Gew.-%

D   - ein hydroxylgruppenhaltiges Amin in 10 bis 20 Gew.-%

E   - ein Fotoinitiator in 1 bis 5 Gew.-%.

Die nach der Erfindung hergestellten Hydrogele zeichnen sich durch gute Haftung am Applikationsort, eine ausgezeichnete Flüssigkeits-Absorptionsfähigkeit, gute mechanische Eigenschaften und zufriedenstellende Transparenz aus.

Die nach der Erfindung für die Photopolymerisation geeigneten Ausgangsgemische enthalten eine Vinylcarbonsäure, die aufgrund ihrer Hydrophilie durch die vorhandenen Carboxylgruppen eine ausreichende Wundsekretaufnahme garantieren.

Neben der Acryl- und Methacrylsäure seien als Beispiele für die Herstellung von Hydrogelen mit den geforderten Eigenschaften als geeignete Säuren $\beta$-Acryloyloxypropionsäure, Vinylessigsäure, Aconitsäure, Trichloracrylsäure, Dimethylacrylsäure, Crotonsäure, Fumarsäure, Zimtsäure und Itaconsäure angeführt. Davon haben sich die Acryl-, Methacryl- und $\beta$-Acryloyloxypropionsäure besonders bewährt.

Die polymerisierbaren Ausgangsgemische enthalten weiterhin olefinisch ungesättigte Monomere, welche vorzugsweise aus der Familie der Acrylate stammen. Damit sind Derivate gemeint, die sich von der Acrylsäure durch Substitution in der 1- und/oder 2-Stellung herleiten lassen. Zur Herstellung selbstklebender Hydrogele sind alle derartigen Derivate geeignet, die zu Homo- oder Copolymerisaten führen, die eine relativ niedrige Glasübergangstemperatur besitzen. Bevorzugt sind Ester der Acrylsäure und Methacrylsäure, von denen beispielsweise Methyl-, Ethyl-, Propyl-, Isopropyl-, Butyl-, Isobutyl-, Hexyl-, Heptyl-, n-Octyl-, Isooctyl-, 2-Ethylhexyl-, 2-Methylheptyl-, Nonyl-, Isononylacrylat, Decyl-, Isodecyl- oder Dodecyl(meth)-acrylat genannt seien.

Um den Hydrogelen weiche und elastische Eigenschaften zu verleihen und ihre Hydrophilie zu steigern, enthalten die Ausgangsgemische Polyoxalkylene mit einer Molekularmasse bis 2000 Dalton, Glycerin oder deren Ester.

Unter den Polyoxyalkylenen werden Polyoxyethylene, Polyoxypropylene und Copolymerisate aus Ethylenoxid und Propylenoxid mit einer Molekularmasse bis 2000 Dalton eingesetzt. Vorzugsweise werden Polyethylenglykol 200, Polyethylenglykol 300, Polyethylenglykol 400, Polyethylenglykol 600, Polypropylenglykol 400 und Polypropylenglykol 1020 eingesetzt.

Glycerin und ihre Ester haben sich aufgrund ihrer guten physiologischen Verträglichkeit besonders gut bewährt.

Diese Polyoxyalkylene sowie Glycerin und ihre Ester sind praktisch nicht flüchtig, ein Vorteil, der im Hinblick auf ihre Verwendung als Weichmacher und Feuchthaltemittel von großer Bedeutung ist.

Zwecks Verbesserung der mechanischen Eigenschaften und Wasseraufnahmefähigkeit des Hydrogels durch Neutralisation der Carboxylgruppen der Vinylcarbonsäure bei der Reaktion zwischen Carboxyl- und Aminogruppen enthält das polymerisationsfähige, lösemittelfreie Ausgangsgemisch ein hydroxylgruppenhaltiges Amin, das aus der Gruppe Diethanolamin, Triethanolamin, N-Methyldiethanolamin, Triisoethanolamin, 2-Amino-2-methyl-1,3-propandiol, Trishydroxymethylaminomethan oder Dimethylethanolamin besteht.

Da die in der Zubereitung enthaltenen C = C-Doppelbindungen nicht direkt durch UV-Strahlen aktiviert werden können, ist die Anwesenheit von mindestens einem Photoinitiator zwingend. Diese Verbindungen sind in der Lage, UV-Strahlenenergie unter Bildung von Radikalen aufzunehmen und damit die Polymerisationsreaktion zu starten. Zur Durchführung der Erfindung ist es erforderlich, daß der Photoinitiator bei der Emissionswellenlänge des benutzten Lasers ein Absorptionsvermögen besitzt, d.h. die Auswahl der Photoinitiatoren muß in Abhängigkeit vom Lasertyp getroffen werden. Für den Wellenlängenbereich von 180-400 nm sind zahlreiche Photoinitiatoren bekannt, die beispielsweise folgenden Gruppen angehören: Acetophenone, Benzophenone, Hydroxyalkylphenone, $\alpha$-Acyloximester, $\alpha$-Halogenketone, Thioxanthone, Fluorenon-Derivate und Michlers Keton.

Durch die Verwendung der monochromatischen Strahlung eines Lasers beim Start der Polymerisation ist es möglich, in das erfindungsgemäß zusammengesetzte zu polymerisierende Ausgangsgemisch bei einer einzigen Wellenlänge in einem Maße Energie einzustrahlen, wie es bei Verwendung konventioneller UV-Lampen nicht gegeben ist. Da andererseits die Geschwindigkeit der Polymerisation unter anderem von der Höhe dieser Initialenergie abhängt, stellt die Laser-initiierte Polymerisation ein Verfahren zur Herstellung von Haftklebern dar, das bezüglich der technologischen Umsetzung Nachteile des Standes der Technik überwindet. In der Publikation "Lasers and Photopolymers" von C. Carre et al. Laser Chem. 10, (1990), 349-366 wird ein Überblick über die laserinduzierte Polymerisation gegeben. Danach sind auch schon Derivate der Acrylsäure untersucht worden, wobei vornehmlich mehrfunktionelle Monomere zur Härtung von Oberflächenbeschichtungen zum Einsatz gekommen sind. Eine Massepolymerisation zur Herstellung von Haftklebern wird nicht erwähnt.

Die erfindungsgemäß einzusetzenden Laser geben ihre Strahlen im Wellenlängenbereich von 180 bis 400 nm ab. Ihre Energieabgabe ist dabei kontinuierlich oder geschieht in gepulster Form. Als kontinuierliche Laser sind beispielsweise die Ionen-Krypton/Argon-Laser (ca. 350 nm) und der Ionen-Helium-Cadmium-Laser (325 nm) zu nennen. Die gepulsten Laser genießen jedoch den Vorzug, daß sie höhere Impulsleistungen im UV-Bereich liefern. Zu ihnen gehören beispielsweise die sogenannten Excimer-Laser, die vornehmlich mit Fluor oder Edelgashalogengemischen betrieben werden. Als typische Vertreter seien die ArF- (193 nm)-, KrF- (248 nm)-, XeCl- (308 nm) und XeF-(351 nm)-Laser genannt.

Auch der gepulste Nd : YAG-Laser kann hier eingesetzt werden, da seine Emission durch Frequenzverdopplung in den UV-Bereich (266 und 365 nm) verschoben werden kann. Eine weiterführende Tabelle 5 über Gas-Laser im Bereich 180-400 nm ist im "Handbook of Laser Science and Technology" Vol. 2, Seite 497-500, CRC Press, Boca Reton 1985, enthalten, auf die her ausdrücklich Bezug genommen wird. Die oben angegebenen Zahlen in Klammern stellen die jeweilige Emissionswellenlänge dar.

Die Pulsdauer bei diesen Laser-Typen liegt im Bereich unterhalb von 50 milliardstel Sekunden (50 Nanosekunden), wobei Pulsfolgefrequenzen bis zu mehreren Hundert Hz einstellbar sind. Die Gesamtzahl der emittierten Pulse ist programmierbar. Die mittlere Leistung dieser Laser kann bei Werten bis zu 100 Watt liegen. Im Pulsbetrieb werden allerdings Leistungen von ca. 20 Megawatt erreicht, woraus sich Leistungsdichten von über einem Gigawatt pro Quadratzentimeter ($10^9$ Watt/cm$^2$) ergeben. Die erreichbare Pulsenergie ist mit mehreren Hundert mJoule um ein Vielfaches höher als die Strahlungsenergie einer UV-Lampe bei der entsprechenden Wellenlänge. Die in diesem Zusammenhang für UV-Lampen angegebenen Energiewerte beziehen sich immer auf den gesamten Spektralbereich und nicht auf eine einzelne Wellenlänge.

Zur Durchführung des erfindungsgemäßen Verfahrens, das vorzugsweise kontinuierlich gestaltet wird, kann die fließfähige Zubereitung vollflächig oder auch musterförmig beispielsweise durch Rakeln, Rollen, Walzen oder mit Hilfe einer Düse auf einer Unterlage in der gewünschten Schichtdicke verteilt werden. Die Schichtdicken liegen dabei zwischen 50 und 5000 $\mu$m und vorzugsweise zwischen 100 und 3000 $\mu$m. Als Unterlage sind alle Flächengebilde geeignet, deren Oberfläche das Ablösen der hergestellten, selbstklebenden Hydrogele erlaubt. Bei der Wahl des Materials der vorzugsweise bahnförmigen Unterlage kann beispielsweise auf Polymere wie Polyethylen, Polypropylen, Polyester und Polyamide oder auf Papier zurückgegriffen werden, wenn ihre Oberfläche zum Beispiel durch Siliconisierung klebstoffabweisend eingestellt sind. Daneben kann die Unterlage auch aus Metallen oder textilen Flächengebilden bestehen. Ohne besondere Behandlung sind auch Polytetraflurethylen, Polyvinylchlorid und mit strahlenvernetztem Perfluorvinylether beschichtete Polyesterfolien geeignet.

Die so erzeugte Schicht der Zubereitung wird dann der monochromatischen Strahlung des Lasers ausgesetzt, wobei die Unterlage und der Laserstrahl relativ zueinander verschiebbar sein können. So kann der Laserstrahl beispielsweise durch Schwenkbewegungen senkrecht zur Fortbewegungsrichtung einer Unterlagenbahn die Polymerisation in der gesamten Fläche auslösen. Die dem Laser eigene punktförmige Konzentration seiner Strahlungsenergie erfordert bei einem örtlich fixierten Laserstrahl und einer sich darunter hinwegbewegenden Unterlagenbahn besondere Verfahrensmaßnahmen. Sie bestehen in der Anbringung optischer Vorrichtungen in dem Laserstrahl, durch die in mindestens einer Richtung eine Aufspreizung des Laserstrahls bevorzugt senkrecht zur Bewegungsrichtung der Unterlagenbahn bewirkt wird. Natürlich hat diese Aufspreizung eine Reduktion der Energiedichte in der bestrahlten Fläche zur Folge, doch werden aufgrund der hohen Energie des Primärstrahls, die ja eine der besonderen Eigenschaften des Lasers darstellt, die für die Polymerisationsauflösung notwendigen Energiedichten von 0,1 bis 320 mJoule/cm$^2$ selbst bei hohen Spreizungswinkeln noch erreicht. Anzumerken ist, daß die Bestrahlung bei Zimmertemperatur erfolgt. Natürlich kann die Temperatur auch davon abweichen, was aber keine besonderen Vorteile bringt. Im Zuge der Polymerisation kommt es zu einer Erwärmung der polymerisierenden Schicht. Diese führt jedoch nicht zu negativen Nebeneffekten. Da die bei den UV-Lampen üblichen IR-Strahlungsanteile fehlen, kann in den meisten Fällen auf eine Kühlung verzichtet werden. Besondere Beachtung ist allerdings dem Sauerstoffgehalt der Luft zu schenken, da das Sauerstoffmolekül bekanntermaßen mit Radikalen in einer Geschwindigkeit reagiert, die je nach Monomer 10.000 bis 1.000.000-mal schneller ist als die eigentliche Polymerisationsreaktion. Außerdem absorbiert der Sauerstoff UV-Strahlen unterhalb von 200 nm. Der Ausschluß von Sauerstoff durch Wahl einer inerten Atmosphäre, wie z.B. Stickstoff, oder durch Vakuum ist daher immer dann angezeigt, wenn kurze Reaktionszeiten angestrebt werden oder die Bestrahlung bei einer Wellenlänge unterhalb 200 nm durchgeführt werden soll. Ein brauchbarer Schutz in dieser Richtung kann auch durch Abdeckung des zu bestrahlenden Substrats mit einer UV-durchlässigen Folie erreicht werden.

Bei einer bevorzugten Realisierung des erfindungsgemäßen Verfahrens ist die Unterlage bahnförmig ausgelegt, so daß die mögliche Bahngeschwindigkeit den Umfang des in der Zeiteinheit produzierten selbstklebenden Hydrogelmaterials festlegt. Die Bahngeschwindigkeit ist von einer Reihe von Parametern abhängig, von denen die Zusammensetzung der Zubereitung, die Schichtdicke der Zubereitung und die Bestrahlungszeit den größten Einfluß haben. Da mit den gepulsten Lasern wirksame Bestrahlungszeiten unter einer Sekunde möglich sind, andererseits dickere Zubereitungsschichten zur Polymerisation längere Bestrahlungszeiten benötigen, liegen die Zeiten bei dem erfindungsgemäßen Verfahren bei $10^{-9}$ bis $10^3$ Sekunden mit einem bevorzugten Bereich von $10^{-2}$ bis $3 \cdot 10^2$ Sekunden. Die produktionsmäßig für die kontinuierliche Fahrweise interessantesten Bestrahlungszeiten liegen unter 1 Sekunde, da sich dann Bahngeschwindigkeiten im Meter-Bereich ergeben. Nicht zuletzt besteht die Möglichkeit zur Erhöhung dieser Geschwindigkeit durch gleichzeitigen Einsatz mehrerer Laser, die dieselbe oder auch verschiedene Emissionswellenlängen aufweisen. Eine räumlich versetzte Doppelbestrahlung läßt sich auch durch Aufteilung des Primärstrahlers in zwei Teilstrahler realisieren, die in festgelegtem Abstand auf die Zubereitungsschicht gelenkt werden.

Die längeren Bestrahlungszeiten über 1 Sekunde kommen vor allem bei einer diskontinuierlichen Ausführung des Verfahrens in Betracht.

Ist die schichtförmige Zubereitung auf einer Unterlage verteilt, die für UV-Strahlen durchlässig ist, so kann die Bestrahlung auch von der Unterseite der Unterlage her erfolgen, wozu der Laserstrahl mit Hilfe einer Glasfaseroptik in die geeignete Position gebracht werden kann. Diese Bestrahlung kann vor, während oder nach der Bestrahlung auf der Oberseite erfolgen. Besondere vorteile dieser Verfahrensvarianten liegen bei der Herstellung von sehr dicken Haftkleberschichten.

Bei der Wahl der Verfahrensparameter sind einige Grundregeln zu beachten, um das gewünschte Resultat zu erzielen:

- Je kürzer die Emissionswellenlänge des Lasers, umso größer die Polymerisationsgeschwindigkeit.
- Je größer die Anzahl der Pulse des Lasers, umso höher der Umsatz.
- Je niedriger die Pulsfolgefrequenz, umso größer die Molekularmasse des Endproduktes.

Ein weiterer Hinweis liegt in der Anmerkung, daß die dem Monomeren üblicherweise zugesetzten Polymerisationsinhibitoren vor der erfindungsgemäßen Polymerisation nicht entfernt zu werden brauchen.

Nach Abschluß der mittels Laserstrahlen induzierten Polymerisationsreaktion wird das entstandene flächige selbstklebende Hydrogelmaterial in dem Falle, daß die Unterlage ein umlaufendes endloses Förderband ist, von diesem auf eine andere wieder ablösbare Unterlage transferiert und der gewünschten Konfektionierung zugeführt.

Die Erfindung wird anhand der folgenden Beispiele weiter erläutert:

In allen Beispielen kommt der Excimer-Laser Typ LPX 210 (Lambda-Physic) mit folgenden Kenngrößen zum Einsatz:

| Emissionswellenlänge | 351 nm |
|---|---|
| Maximale Durchschnittsleistung | 28 W |
| Maximale Pulsenergie | 320 mJ |
| Pulsdauer | 30 ns |
| Maximale Pulsfolgefrequenz | 100 Hz |

ABKÜRZUNGSVERZEICHNIS

| | |
|---|---|
| AS | - Acrylsäure |
| MAS | - Methacrylsäure |
| APS | - $\beta$-Acryloyloxypropionsäure |
| BA | - Butylacrylat |
| 2-EHA | - 2-Ethylhexylacrylat |
| IO | - Isooctylacrylat |
| PEG 300 | - Polyethylenglykol mit einer Molekularmasse von 300 Dalton |
| PPG 400 | - Polypropylenglykol mit einer Molekularmasse von 400 Dalton |
| GN | - Glycerin |
| DEA | - Diethanolamin |

TEA         - Triethanolamin
TIEA       - Triisoethanolamin
ZLI 3331   - 4(2-Acryloylethoxy)-phenyl-(2-hydroxy-2-propyl)-keton
ABP        - 4-Acryloyloxybenzophenon
PAC        - Phenyl-(1-acryloyloxy)-cyclohexylketon

Beispiel 1

Ein Ausgangsgemisch aus 50 Gew.-% Acrylsäure, 18 Gew.-% Butylacrylat, 15 Gew.-% Polyethylenglykol 300, 15 Gew.-% Diethanolamin und 2 Gew.-% 4(2-Acryloylethoxy)-phenyl-(2-hydroxy-2-propyl)-keton wird bei Raumtemperatur in eine mit planarem Boden versehene Schale aus Polytetrafluorethylen (PTFE) gegeben, so daß die aufgetragene Schichtdicke 1000 $\mu$m beträgt. Das Ausgangsgemisch wird einer Laserbestrahlung mit folgenden Arbeitsparametern unterworfen:

| | |
|---|---|
| Angewandte absolute Energie | 160 mJ |
| Angewandte Pulsfolgefrequenz | 5 Hz |
| Energiedichte | 35,6 mJ/cm$^2$ |
| Pulsenzahl | 400 |
| Bestrahlungszeit | 80 s |

Das daraus resultierende selbstklebende Hydrogel wird aus der Schale genommen und zur weiteren Untersuchung der Wasseraufnahme verwendet.

Beispiele 2 bis 15

Auf analoge Weise werden selbstklebende Hydrogele mit den in der Tabelle 1 aufgeführten Zusammensetzungen erhalten.

Prüfung der Wasseraufnahme

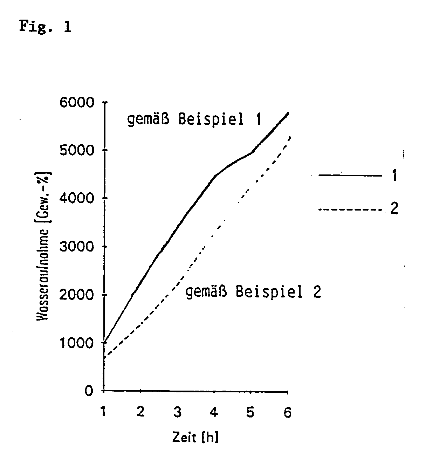

Aus den mittels Laserstrahlen hergestellten Hydrogelen wird ein 2,5 cm x 2,5 cm guadratisches Muster ausgeschnitten und sechs Stunden in destilliertem Wasser bei Raumtemperatur gelagert. Die Wasseraufnahme wird aufgrund der Massenunterschiede zwischen wasserhaltigem Hydrogel und Hydrogel in trockenem Zustand ermittelt. Die Werte der Wasseraufnahme wurden in der Tabelle 2 zusammengefaßt und in Fig. 1 für ausgewählte selbstklebende Hydrogele graphisch dargestellt.

## Tabelle 1

Zusammensetzungen der mittels Laserstrahlen hergestellten selbstklebenden Hydrogele

| Beispiel | Vinylcarbonsäure [Gew.-%] | | | (Meth)acrylsäure-alkylester [Gew.-%] | | | Polyoxyalkylen/ Glycerin [Gew.-%] | | | Hydroxylgruppenhaltiges Amin [Gew.-%] | | | Fotoinitiator [Gew.-%] | | |
|---|---|---|---|---|---|---|---|---|---|---|---|---|---|---|---|
| | AS | MAS | APS | BA | 2-EHA | IO | PEG 300 | PPG 400 | GN | DEA | TEA | TIEA | ZLI 3331 | ABP | PAC |
| 2 | 60 | -- | -- | -- | 10 | -- | 19 | -- | -- | -- | 10 | -- | 1 | -- | -- |
| 3 | 40 | -- | -- | 14 | -- | -- | -- | -- | 30 | -- | 15 | -- | -- | 1 | -- |
| 4 | -- | -- | 30 | 15 | -- | 15 | 10 | -- | 8 | 20 | -- | -- | -- | -- | 2 |
| 5 | 30 | 10 | -- | -- | 30 | -- | -- | 13 | -- | -- | -- | 14 | 2 | 1 | -- |
| 6 | -- | 10 | 15 | -- | 15 | -- | 20 | -- | 20 | -- | 15 | -- | 5 | -- | -- |
| 7 | 40 | -- | -- | -- | -- | 20 | -- | 26 | -- | 12 | -- | -- | 1 | -- | 1 |
| 8 | 30 | -- | -- | 10 | -- | -- | -- | -- | 40 | 10 | -- | 8 | -- | 2 | -- |
| 9 | -- | 10 | 40 | -- | -- | 15 | 15 | -- | -- | -- | 17 | -- | 1,5 | -- | 1,5 |
| 10 | 20 | -- | 10 | 17 | -- | -- | 18 | -- | 20 | 12 | -- | -- | 3 | -- | -- |
| 11 | 35 | -- | -- | -- | 15 | -- | 20 | -- | 12 | -- | 10 | 5 | -- | 3 | -- |
| 12 | -- | 10 | 30 | 29 | -- | -- | -- | -- | 15 | -- | 15 | -- | 1 | -- | -- |
| 13 | -- | -- | 40 | -- | -- | 16 | -- | 30 | -- | 11 | -- | -- | 2 | 1 | -- |
| 14 | 40 | -- | -- | -- | 30 | -- | 14 | -- | -- | -- | 13 | -- | -- | -- | 3 |
| 15 | 25 | -- | 25 | 5 | 10 | -- | 10 | -- | 5 | 18 | -- | -- | 1,5 | 0,5 | -- |

EP 0 670 338 A2

Tabelle 2

| Wasseraufnahme der mittels Laserstrahlen hergestellten selbstklebenden Hydrogelen | | | | | | |
|---|---|---|---|---|---|---|
| Beispiel | Wasseraufnahme [Gew.-%] nach ....... | | | | | |
| | 1h | 2h | 3h | 4h | 5h | 6h |
| 1 | 986 | 2303 | 3458 | 4477 | 4971 | 5819 |
| 2 | 673 | 1405 | 2233 | 3293 | 4268 | 5120 |
| 3 | 813 | 1916 | 2830 | 3866 | 4512 | 5482 |
| 4 | 780 | 1830 | 2010 | 3335 | 4290 | 5188 |
| 5 | 762 | 1750 | 1980 | 3066 | 4012 | 4962 |
| 6 | 750 | 1700 | 1830 | 2860 | 3844 | 4725 |
| 7 | 948 | 2216 | 3337 | 4268 | 4863 | 5728 |
| 8 | 886 | 2116 | 3216 | 4198 | 4711 | 5614 |
| 9 | 880 | 2194 | 3311 | 4216 | 4768 | 5681 |
| 10 | 916 | 2086 | 3216 | 4322 | 4819 | 5632 |
| 11 | 942 | 2223 | 3412 | 4422 | 4912 | 5689 |
| 12 | 813 | 1986 | 3028 | 4001 | 4316 | 5217 |
| 13 | 771 | 1812 | 2788 | 3844 | 4211 | 5083 |
| 14 | 863 | 2002 | 2993 | 3980 | 4719 | 5556 |
| 15 | 808 | 1910 | 2717 | 3784 | 4526 | 5461 |

**Patentansprüche**

1. Verfahren zur Herstellung von selbstklebenden Hydrogelen durch radikalische Polymerisation eines photopolymerisationsfähigen, lösemittelfreien Ausgangsgemisches mit Hilfe von Ultraviolettstrahlen im Bereich von 180 - 400 nm, wobei Polymerisationsauslösung erforderliche Energie durch monochromatische Strahlung eines Lasers zugeführt wird, dadurch gekennzeichnet, daß das polymerisationsfähige, lösemittelfreie Ausgangsgemisch folgende Bestandteile enthält:
   A    - eine Vinylcarbonsäure
   B    - ein (Meth)acrylsäurealkylester mit 1 bis 12 Kohlenstoffatomen im Alkylrest
   C    - ein Polyoxyalkylen mit einer Molekularmasse bis 2000 Dalton, Glycerin oder deren Ester
   D    - ein hydroxylgruppenhaltiges Amin
   E    - einen Fotoinitiator.

2. Verfahren nach Anspruch 1, dadurch gekennzeichnet, daß die Bestandteile des Ausgangsgemisches bevorzugt in folgender Konzentration eingesetzt werden:
   A    - eine Vinylcarbonsäure in 30 bis 60 Gew.-%
   B    - ein (Meth)acrylsäurealkylester mit 1 bis 12 Kohlenstoffatomen im Alkylrest in 10 bis 30 Gew.-%
   C    - ein Polyoxyalkylen mit einer Molekularmasse bis 2000 Dalton, Glycerin oder deren Ester in 10 bis 40 Gew.-%
   D    - ein hydroxylgruppenhaltiges Amin in 10 bis 20 Gew.-%
   E    - ein Fotoinitiator in 1 bis 5 Gew.-%.
wobei die gesamte Konzentration an Bestandteilen A + B + C + D + E = 100 Gew.-% beträgt.

3. Verfahren nach den Ansprüchen 1 oder 2, dadurch gekennzeichnet, daß der Vinylcarbonsäure aus der Gruppe ausgewählt ist, die aus der Acrylsäure, β-Acryloyloxypropionsäure, Vinylessigsäure, Itaconsäure, Methacrylsäure, Aconitsäure, Fumarsäure, Trichloracrylsäure und Dimethylacrylsäure besteht.

4. Verfahren nach den Ansprüchen 1 oder 2, dadurch gekennzeichnet, daß (Meth)acrylsäurealkylester mit 1 bis 12 Kohlenstoffatomen im Alkylrest aus der Gruppe ausgewählt ist, die aus Methyl-, Ethyl-, Propyl-, Isopropyl-, Butyl-, Isobutyl-, Hexyl-, Heptyl-, 2-Ethylhexyl-, 2-Methylheptyl-, Isooctyl-, Nonyl-, Isononyl-, Decyl-, Isodecyl- oder Dodecyl(meth)-acrylat besteht.

5. Verfahren nach den Ansprüchen 1 oder 2, dadurch gekennzeichnet, daß das Polyoxyalkylen mit einer Molekularmasse bis 2000 Dalton ein Polyoxyethylen, Polyoxypropylen oder ein Copolymerisat aus Ethylenoxid und Propylenoxid ist.

6. Verfahren nach einem oder mehreren der Ansprüche 1 bis 5, dadurch gekennzeichnet, daß ein hydroxylgruppenhaltiges Amin ein ethoxyliertes Amin ist oder aus der Gruppe Diethanolamin, Triethanolamin, N-Methyldiethanolamin, Triisoethanolamin, 2-Amino-2-methyl-1,3-propandiol, Trishydroxymethylaminomethan oder Dimethylethanolamin besteht.

**7.** Verfahren nach den Ansprüchen 1 oder 2, <u>dadurch gekennzeichnet,</u> daß der Fotoinitiator aus der Gruppe der Acetophenone, Benzophenone, Benzil-Derivate, Benzoin-Derivate, Dialkoxyacetophenone, Hydroxyalkylphenone, $\alpha$-Acyloximester, $\alpha$-Halogenketone, Thioxanthone, Fluorenon-Derivate oder Michlers Keton ausgewählt ist.

**8.** Verfahren nach Anspruch 1, <u>dadurch gekennzeichnet,</u> daß ein gepulster Laser eingesetzt wird.

**9.** Verfahren nach Anspruch 1, <u>dadurch gekennzeichnet,</u> daß der Laserstrahl eine Energiedichte von 0,1-320 mJoule/cm$^2$ erzeugt.

**10.** Verfahren nach einem oder mehreren der vorangehenden Ansprüche, <u>dadurch gekennzeichnet,</u> daß das in einer Dicke von 50 bis 5000 $\mu$m flächig verteilte Ausgangsgemisch der monochromatischen Laserstrahlung ausgesetzt wird.

**11.** Verfahren nach einem oder mehreren der vorangehenden Ansprüche, <u>dadurch gekennzeichnet,</u> daß die Dauer der UV-Laserbestrahlung in dem Bereich von $10^{-9}$ bis $10^3$ Sekunden liegt.

**12.** Verfahren nach Anspruch 11, <u>dadurch gekennzeichnet,</u> daß die Dauer im Bereich von $10^{-2}$ bis $3 \times 10^2$ Sekunden liegt.

**13.** Verfahren nach einem oder mehreren der vorangehenden Ansprüche, <u>dadurch gekennzeichnet,</u> daß die UV-Bestrahlung unter Ausschluß von Sauerstoff ausgeführt wird.

**14.** Verfahren nach einem oder mehreren der vorangehenden Ansprüche, <u>dadurch gekennzeichnet,</u> daß die Polymerisation kontinuierlich unter Relativbewegung der Laserstrahlen und des Ausgangsgemisches zueinander ausgeführt wird.

**15.** Verfahren nach einem oder mehreren der vorangehenden Ansprüche, <u>dadurch gekennzeichnet,</u> daß das Ausgangsgemisch mit einem Laserstrahl bestrahlt wird, der mittels einer optischen Einrichtung in mindestens einer Richtung aufgespreizt ist.

**16.** Verfahren nach einem der mehreren der vorangehenden Ansprüche, <u>dadurch gekennzeichnet,</u> daß zur Durchführung der Polymerisation mehrere Laser gleichzeitig oder nacheinander eingesetzt werden und diese die gleiche oder verschiedene Emissionswellenlängen aufweisen.

**18.** Verwendung der selbstklebenden Hydrogele nach Ansprüchen 1 bis 16 in unsteriler oder steriler Form als Wundauflage.

Fig. 1